# EUROPÄISCHE PATENTANMELDUNG

(11) **EP 0 537 533 A1**
(43) Veröffentlichungstag der Anmeldung: **21.04.1993**
(21) Anmeldenummer: 92116500.7
(22) Anmeldetag: 26.09.1992
(51) Int. Cl.: A61B 19/00, A61B 17/28

(54) **Vorrichtung zur laparoskopischen Nephrektomie**

(30) Priorität: 15.10.1991 DE 4134029
(71) Anmelder: ANGIOMED AG, D-76227 Karlsruhe (DE)
(72) Erfinder: Schnepp-Pesch, Wolfram, W-7500 Karlsruhe 41 (DE); Lindenberg, Josef, W-7500 Karlsruhe 41 (DE)
(74) Vertreter: Dipl.-Ing. Heiner Lichti Dipl.-Phys. Dr.rer.nat. Jost Lempert Dipl.-Ing. Hartmut Lasch

(57) **Zusammenfassung**

Die Erfindung schlägt zur Ermöglichung einer laparoskopischen Nephrektomie eine entsprechende Vorrichtung vor, welche gekennzeichnet ist durch einen Kescher (1) mit einem Auffangbeutel (11) und einem Auffangrahmen (4) an dessen oberen Rand, wobei der Auffangrahmen aus einem Draht aus Formgedächtnislegierung besteht, der in seiner Hochtemperaturstellung einen Ring bildet, von dem zwei Drahtabschnitte (7) sich parallel zueinander verlaufend forterstrecken und einen Griff des Keschers bilden und durch eine die parallel verlaufenden Drahtabschnitte umgebende stabile Hülse (8).

## Beschreibung

Die Erfindung betrifft eine Vorrichtung zur laparoskopischen Nephrektomie.

Eine Nephrektomie, d.h. die Entfernung oder Exstirpation einer Niere bei einer Schrumpfniere, einem Totalinfarkt einer Niere, einem Tumor oder dergleichen erfolgt in herkömmlicher Weise dadurch, daß über nahezu den halben Teilumfang des Körpers des Patienten ein Öffnungsschnitt gelegt wird, durch welchen die Niere zugänglich wird, so daß sie aus dem sie haltenden umgebenden Gewebe gelöst und entfernt werden kann. Eine derartige Operation ist abgesehen von der Entfernung der Niere an sich für den Patienten mit erheblichen Belastungen verbunden. Die Heilung der Wunde benötigt beträchtliche Zeit. Die Operation kann mit erheblichen Komplikationen verbunden sein. Auch die Nachbehandlung und der Heilungsprozeß der Wunde kann zu Komplikationen führen.

Der Erfindung liegt daher die Aufgabe zugrunde, eine Vorrichtung zu schaffen, die eine laparoskopische Nephrektomie durchzuführen erlaubt.

Erfindungsgemäß wird die genannte Aufgabe durch eine derartige Vorrichtung gelöst, welche gekennzeichnet ist durch einen Kescher mit einem Auffangbeutel und einem Auffangrahmen an dessen oberen Rand, wobei der Auffangrahmen aus einem Draht aus Formgedächtnislegierung besteht, der in seiner Hochtemperaturstellung einen Ring bildet, von dem zwei Drahtabschnitte sich parallell zueinander verlaufend forterstrecken und einen Griff des Keschers bilden und durch eine die parallell verlaufenden Drahtabschnitte umgebende stabile Hülse.

Im Ausgangszustand befindet sich sowohl der Auffangrahmen, der aus Draht aus Formgedächtnislegierung (Memory-metall, wie Nitinol, Bimetall oder dergleichen) gebildet ist, als auch der Beutel innerhalb der Hülse, in die der Auffangrahmen bei einer unterhalb der Umwandlungstemperatur der Formgedächtnislegierung liegenden Temperatur im Herstellerwerk des Herstellers der erfindungsgemäßen Vorrichtung eingebracht werden kann. Die Hülse kann dabei einen Durchmesser aufweisen, der laparoskopischen Gegebenheiten angemessen ist, beispielsweise im Bereich von 10 mm. Der Beutel selbst besteht aus einem Kunststoffmaterial. Zur Sicherheit kann weiterhin vorgesehen sein, daß der Auffangbeutel durch eine Farbenstruktur verstärkt ist, wobei insbesondere die Verstärkungsstruktur aus Drahtfäden gebildet ist. Auch die Verstärkungsstruktur muß sich herstellermäßig zunächst in der Hülse befinden. Damit sie nach Freigabe aus der Hülse innerhalb des Körpers des Patienten ebenfalls selbständig eine der Beutelform angepaßte bogenförmige Kontur annimmt, kann in bevorzugter Ausgestaltung vorgesehen sein, daß der Draht der Verstärkungsstruktur ebenfalls aus Formgedächtnislegierungen besteht. Während der den Auffangrahmen bildende Draht relativ stark ausgebildet sein, damit der Rahmen und die den Griff bildendenden Drahtabschnitte eine hinreichende Biegefestigkeit haben, können die die Verstärkungsstruktur für den Beutel bildenden Drähte äußerst dünn sein und einen Durchmesser von einem Bruchteil eines mm aufweisen, so beispielsweise 0,4 - 0,8 mm oder auch weniger.

Eine äußerst bevorzugte Ausgestaltung sieht vor, daß die Verstärkungsstruktur durch von dem Auffangrahmen bogenförmig und im wesentlichen diagonal zu diesem sternförmig hin und her geführte Drahtabschnitte gebildet ist. Eine solche Struktur des Verstärkungsdrahtes ist in einfacher und bequemer Weise in der Hülse der erfindungsgemäßen Vorrichtung unterbringbar. Alternativ kann auch vorgesehen sein, daß die Verstärkungsstruktur netzartig oder korbartig ausgebildet ist. Während weiterhin in bevorzugter Ausgestaltung vorgesehen ist, daß die Verstärkungsstruktur den Beutel umgibt, kann darüber hinaus hierzu alternativ vorgesehen sein, daß die Verstärkungsstruktur durch das Material des Beutels ummantelt ist.

Weitere Vorteile und Merkmale der Erfindung ergeben sich aus den Ansprüchen und aus der nachfolgenden Beschreibung, in der ein Ausführungsbeispiel der erfindungsgemäßen Vorrichtung zur laparoskopischen Nephrektomie im einzelnen erläutert ist. Dabei zeigt:
- Figur 1a: die erfindungsgemäße Vorrichtung in seitlicher perspektivischer Darstellung;
- Figur 1b: eine Daraufsicht auf den Auffangkorb der erfindungsgemäßen Vorrichtung von oben;
- Figur 2a: die Unterbringung von Auffangrahmen und -korb in einer Einführ- und Arbeitshülse;
- Figur 2b-2d: in schematischer Darstellung den in seine weiteste Stellung aufgespannten Auffangrahmen, den durch nach vorne schieben der Hülse verengten Rahmen und den in der Position der Figur 2c teilweise durch die Haut des Patienten herausgezogenen Auffangkorb mit wieder aufgestelltem Rahmen.

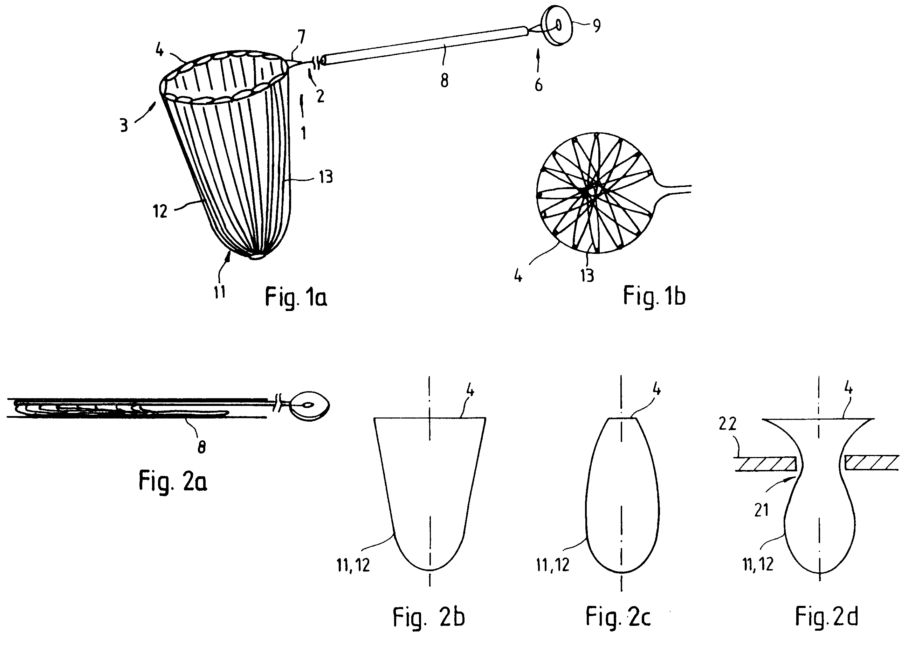

Die erfindungsgemäße Vorrichtung zur laparoskopischen Nephrektomie weist einen Kescher 1 auf. Der Kescher 1 weist einen Draht 2 aus einer Formgedächtnislegierung auf, der mit seinem Mittelteil 3 zu einem Ring in der Hochtemperaturstellung der Formgedächtnislegierung ringförmigen Auffangrahmen 4 gebildet ist, von dem sich zu dem freien Ende 6 Drahtabschnitte 7 parallel zueinander erstrecken. Über die sich parallel zueinander erstreckenden Drahtabschnitte 7 ist eine stabile Hülse 8 geschoben. Diese wird am Heruntergleiten von den Drahtenden 6 durch ein Blockierelement 9, wie einen Ring oder dergleichen gehindert. An dem Auffangrahmen 4 ist ein Beutel 11 befestigt, der aus einer geeigneten dünnen Kunststoffolie besteht. Zur Verstärkung kann der Beutel, wie dies in den Figuren 1a, b, dargestellt ist, mit einer Verstärkungsstruktur 12 versehen sein, die korbähnlich, netzähnlich oder gerippeähnlich ausgebildet sein kann. Die Verstärkungsstruktur 12 besteht aus Fäden, die im dargestellten Ausführungsbeispiel ebenfalls ein Drahtfaden 13 aus Formgedächtnislegierung sind. Der Drahtfaden 13 ist mit einem Ende am Auffangrahmen 4 festgelegt und von dort bogenförmig im wesentlichen diagonal zu der gegenüberliegenden Seite des Auffangrahmens 4 und von dort wieder zurück zur erstgenannten Seite des Auffangrahmens, aber etwas versetzt zu dem Ausgangspunkt geführt etc., so daß in Daraufsicht eine sternähnliche Führung des Drahtes 13 entsteht, wie dies insbesondere in der Figur 1b erkennbar ist.

Der Drahtkorb 12 kann den Kunststoffbeutel umgeben. Das Kunststoffmaterial kann auch die Drahtfäden einschließen und/oder ummanteln.

In der Ausgangsstellung befindet sich auch der Korb 12 mit Beutel 11 innerhalb der Hülse 8, wie dies in Figur 2a gezeigt ist, wobei die einzelnen Drahtabschnitte des Korbes 12 parallel zu den Drahtabschnitten 7 des Auffangrahmens gelegt sind. Während die Drähte des Auffangrahmens 4 und die Drahtabschnitte 2 eine hinreichende Stärke aufweisen können, damit sich eine ausreichende Biegesteifigkeit ergibt, können die Drähte 13 des Korbes 12 äußerst dünn gewählt werden, so daß die Drahtabschnitte 7 und der Korb 12 insgesamt in einer Hülse 8 untergebracht werden können, die noch einen vertretbaren Durchmesser von beispielsweise 10 mm aufweist. Dies ist für einen laparoskopischen Eingriff keine übermäßige Querschnittsabmessung. Das Einbringen von Rahmen 4 und Korb 12 geschieht in der Herstellungsfirma bei unterhalb der Übergangstemperatur der Formgedächtnislegierung gegebener Temperatur und ist dort ohne weiteres durchführbar. Im aufgespannten Zustand weist der Kescherrahmen 4 einen Durchmesser von etwa 70 mm auf. Er hat eine Tiefe von etwa 100 mm. Die Länge der über den Rahmen 4 hinausreichenden Drahtabschnitte liegt bei etwa 300 mm und die Länge der Hülse 8 bei etwa 200 mm, so daß die Korblänge von 100 mm am Behandlungsort voll aus der Hülse 8 herausschiebbar ist.

Die erfindungsgemäße Vorrichtung wird derart eingesetzt, daß sie zunächst mit innerhalb der Hülse 8 angeordnetem Rahmen 4 und Beutel oder Korb 12 perkutan in den die Niere umhüllenden Körperhohlraum eingeführt wird, wobei dieser in an sich bekannter laparoskopischer Weise aufgebläht ist. Innerhalb dieses Körperhohlraums wird der Rahmen 4 und der Beutel 11,12 aus der Hülse 8 herausgeschoben. Der Rahmen 4 stellt sich aufgrund der ihn umgebenden erhöhten Temperatur in der ringförmigen Form mit dem gewünschten Durchmesser von üblicherweise 70 mm auf. Der Kescher wird mit dem Auffangrahmen 4 unter die zu entfernende Niere gebracht. Anschließend wird die Niere durch weitere laparoskopisch vorgesehene Schächte vollständig vom umgebenden Gewebe abgetrennt und fällt schließlich in den Beutel 11 des Keschers 1, von dem sie in der Position der Figur 1a, 1b und 2b aufgefangen wird. Anschließend wird der Durchmesser des ringförmigen Auffangrahmens 4 reduziert, indem die stabile Hülse 8 entlang der Drahtabschnitte 7 bis über den den Rahmen 4 bildenden Bereich geschoben wird, so daß dieser zusammengezogen wird, wie dies in der Figur 2c dargestellt ist. Im folgenden kann der Rahmen 4 durch die Öffnung 21 in der Haut 22 des Patienten nach außen gezogen werden, so daß die Öffnung des Keschers sich außerhalb des Patienten befindet. Anschließend wird der Auffangrahmen 4 wieder freigegeben, so daß er sich wieder in seine weiteste Position aufstellt, wie dies in der Figur 2d dargestellt ist. Da die gesamte Niere nicht durch die doch relativ kleine Öffnung 21 in der Haut des Patienten 22 entfernt werden kann, wird sie innerhalb des Beutels 11, 12 von außen her zerkleinert, beispielsweise durch ein Mixer-ähnliches Gerät oder dergleichen. Anschließend können die zerkleinerten Teile aus dem Beutel 11 abgesaugt werden und dieser schließlich insgesamt durch die Öffnung 21 der Haut 22 des Patienten wieder entnommen werden.

## Patentansprüche

1. Vorrichtung zur laparoskopischen Nephrektomie, gekennzeichnet durch einen Kescher (1) mit einem Auffangbeutel (L) und einem Auffangrahmen (4) an dessen oberen Rand, wobei der Auffangrahmen aus einem Draht (2) aus Formgedächtnislegierung besteht, der in seiner Hochtemperaturstellung einen Ring bildet, von dem zwei Drahtabschnitte (7) sich parallel zueinander verlaufend forterstrecken und einen Griff des Keschers bilden und durch eine die parallel verlaufenden Drahtabschnitte (7) umgebende stabile Hülse (8).

2. Vorrichtung nach Anspruch 1, dadurch gekennzeichnet, daß der Auffangbeutel (L) durch eine Verstärkungsstruktur (12) verstärkt ist.

3. Vorrichtung nach Anspruch 2, dadurch gekennzeichnet, daß die Verstärkungsstruktur (12) aus Drahtfäden gebildet ist.

4. Vorrichtung nach Anspruch 2 oder 3, dadurch gekennzeichnet, daß der Draht (13) der Verstärkungsstruktur (12) ebenfalls aus Formgedächtnislegierung besteht.

5. Vorrichtung nach einem der Ansprüche 2 bis 4, dadurch gekennzeichnet, daß die Verstärkungsstruktur durch von dem Auffangrahmen (4) bogenförmig und im wesentlichen diagonal zu diesem sternförmig hin und her geführte Drahtabschnitte gebildet ist.

6. Vorrichtung nach einem der Ansprüche 2 bis 4, dadurch gekennzeichnet, daß die Verstärkungsstruktur netzartig ausgebildet ist.

7. Vorrichtung nach einem der Ansprüche 2 bis 4, dadurch gekennzeichnet, daß die Verstärkungsstruktur korbartig ausgebildet ist.

8. Vorrichtung nach einem der Ansprüche 2 bis 7, dadurch gekennzeichnet, daß die Verstärkungsstruktur (12) den Beutel (11) umgibt.

9. Vorrichtung nach einem der Ansprüche 2 bis 7, dadurch gekennzeichnet, daß die Verstärkungsstruktur (12) durch das Material des Beutels (11) ummantelt ist.
